# EUROPEAN PATENT SPECIFICATION

(11) **EP 4 157 618 B1**
(45) Date of publication and mention of the grant of the patent: **29.05.2024**
(21) Application number: 21729955.1
(22) Date of filing: 27.05.2021
(51) Int. Cl.: B29D 11/00, A61L 12/08, B08B 11/02

(54) **CARRIER FOR CARRYING AN OPHTHALMIC LENS DURING ITS TREATMENT IN A BATH**
TRÄGER ZUM TRAGEN EINER OPHTHALMISCHEN LINSE WÄHREND DER BEHANDLUNG IN EINEM BAD
SUPPORT POUR PORTER UNE LENTILLE OPHTALMIQUE LORS DE SON TRAITEMENT DANS UN BAIN

(30) Priority: 29.05.2020 US 202063032253 P
(43) Date of publication of application: 05.04.2023
(73) Proprietor: Alcon Inc., 1701 Fribourg (CH)
(72) Inventor: LEIBOLD, Thomas, 63868 Grosswallstadt (DE); SCHMITT, Jennifer, 63868 Grosswallstadt (DE); STRUCKMEIER, Katrin Sylke, 63868 Grosswallstadt (DE); STUTZ, Michael, 63868 Grosswallstadt (DE)
(74) Representative: Bohest AG
(86) International application number: PCT/IB2021/054654
(87) International publication number: WO 2021/240438

(56) References cited:
- CN-A- 101 596 789
- US-A1- 2018 281 321

## Description

### FIELD OF THE INVENTION

The present invention relates to a carrier for carrying an ophthalmic lens during its treatment in a bath. The invention, in other aspects, also relates to a carrier array for forming a plurality of such carriers, to a carrier tray comprising a frame and one or more carrier arrays attached to the frame, and to a carrier stack system comprising a plurality of such carrier trays arranged one above the other to form a stack.

### BACKGROUND OF THE INVENTION

Mass production of ophthalmic lenses, in particular contact lenses such as soft contact lenses, generally includes one or more bath treatments in which the lenses are treated with water or specific treatment liquids in order to modify the properties of the lenses. For example, in the manufacture of silicone hydrogel soft contact lenses a lens-forming material is dispensed into a mold for single use or into a reusable mold and is subsequently cured to form the contact lens. After curing, extractables still contained in the cured contact lens need to be extracted before the contact lens can be worn on the eye. In addition, it may be necessary or desirable to apply a coating to the extracted contact lens in order to increase the contact lens' hydrophilic properties. Both, the extraction process as well as the coating process, are typically performed by successively transporting the cured contact lenses still containing the extractables through one or more baths.

For transporting the contact lenses through the baths, a variety of contact lens carriers have already been proposed. A known embodiment of such a contact lens carrier has the shape of a more or less cylindrical tube with a bottom. The tube and the bottom have openings or slots through which the treatment liquid may enter and exit the interior of the tube to allow the contact lens contained in the interior of the tube to be sufficiently exposed to the treatment liquid. The contact lenses are retained in the interior of such a tube by means of a circular diaphragm having inwardly extending flexible fins, so that once the contact lens has been inserted into the interior of the tube in the space between the diaphragm and at the bottom of the tube, the lens cannot escape from that space until it is removed again from this space, for example with the aid of a suitable gripper.

Another type of carrier for contact lenses is suggested in WO 2018/185630 (preamble of claim 1). The carrier comprises a plurality of baskets with a basket wall defining a concave cavity for accommodating the ophthalmic lenses to be treated. The basket further comprises three or four recesses arranged in the basket wall at different locations along the circumference of the basket, and a retainer comprising retainer arms arranged in a star-shaped configuration corresponding to the arrangement of the recesses along the circumference of the basket.

For extracting the extractables still contained in the cured contact lenses from the lens-forming process, the contact lenses are treated in aqueous baths for removing these exctractables from the cured contact lenses. However, extraction of the cured contact lenses in aqueous baths is comparatively time-consuming.

Alternatively, treatment baths containing special organic solvents have been suggested. And while such organic solvent baths may result in a considerably increased extraction speed when compared to aqueous baths, the contact lenses treated in such baths tend to very substantially swell during treatment in the organic solvent, and to very substantially shrink again upon subsequent treatment in an aqueous bath. The carriers disclosed in WO 2018/185630 are not suitable for such treatment in organic solvents in which the cured contact lenses swell to a substantial extent since they do not offer sufficient space for the substantially increased volume of the swollen contact lenses. Using such carrier may result in that a contact lens may either get compressed/damaged or folded, and may retain this latter state during subsequent shrinking in the aqueous bath so that after completion of the treatment the contact lens may not be automatically removed from the basket of the carrier with the aid of a gripper. Yet further, due to the extraordinary deformability of such contact lens, the contact lens may roll up like a cigar and may even escape from the carrier through the basket wall segments or through the recesses between such basket wall segments such that the contact lens is lost in the bath.

Hence, for reasons of efficiency and cost-effectiveness there is a continuing need for carriers allowing contact lenses to be exposed to a broader range of treatment liquids. Further, it is crucial that the contact lenses are sufficiently well exposed to the treatment liquid as the treatment of the contact lenses, for example extraction of extractables from the cured contact lenses, must be as effective and complete as possible. At the same time, it is necessary to ensure that the contact lenses cannot escape from the carrier during treatment in the bath, and can be reliably removed from the basket of the carrier with the aid of the gripper after treatment is completed.

### SUMMARY OF THE INVENTION

These and other objects are met by a carrier as it is specified by the features of the independent claim 1 directed to such a carrier. Advantageous embodiments of the carrier according to the invention are the subject of the dependent claims. According to one aspect of the invention, a carrier is suggested for carrying of an ophthalmic lens, in particular a contact lens such as a soft contact lens, during its treatment in a bath. The carrier comprises:
a basket comprising a basket wall defining a concave cavity for accommodating an ophthalmic lens, the basket further comprising a plurality of recesses arranged in the basket wall at different angular locations along the circumference of the basket, where each recess of the plurality of recesses is arranged to extend downwardly from an upper rim of the basket wall;
and a retainer comprising a plurality of retainer arms corresponding to the plurality of recesses arranged in the basket wall, the retainer arms being arranged in a star-shaped configuration at angular locations corresponding to the angular locations of the recesses along the circumference of the basket, to in an assembled state of two such carriers extend into a corresponding one of the plurality of recesses in the basket wall to retain the ophthalmic lens in the carrier. The basket wall defining the concave cavity comprises first and second concavely curved sections, the first and second concavely curved sections each having a curvature, and the curvature of the first concavely curved section is different from the curvature of the second concavely curved section.

According to one aspect of the carrier according to the invention, the first concavely curved section is a lower section and the second concavely curved section is an upper section of the basket wall, the upper section and the lower section being coaxially arranged about a central axis of the basket.

According to a further aspect of the carrier according to the invention, the lower section has a stronger curvature than the upper section.

In accordance with still a further aspect of the carrier according to the invention, the retainer arms are arranged to have a protruding shape to in the assembled state of two such carriers protrude into the concave cavity defined by the basket wall to further limit a space for the ophthalmic lens in the carrier.

In accordance with yet a further aspect of the carrier according to the invention, the protruding shape comprises first and second portions of the retainer arms, the first portion having a convex shape and the second portion having a concave shape.

According to still a further aspect of the carrier according to the invention, the retainer arms radially merge in a common central hub.

According to yet a further aspect of the carrier according to the invention, the basket wall comprises a transition section arranged between and connecting the first and second concavely curved sections of the basket wall, wherein the transition section connecting the first and second concavely curved sections is rounded to connect the first and second concavely curved sections without forming any sharp edges.

In accordance with another aspect of the carrier according to the invention, the basket wall comprises a plurality of basket wall segments, in particular at least eight basket wall segments, which are adjacently arranged to one another in the direction of the circumference of the basket. Each individual basket wall segment of the plurality of basket wall segments is formed by a centrally arranged common basket wall bottom and by two adjacently arranged webs extending radially outwardly from the common basket wall bottom to a radial outer end of the respective web. The radial outer end of the respective web is arranged in one of the recesses of the plurality of recesses, and the individual basket wall segment is further formed by a rim portion connecting the radial outer ends of the two adjacently arranged webs to define an opening bounded by the common basket wall bottom, the two adjacently arranged webs and the rim portion.

In accordance with a further aspect of the carrier according to the invention, the basket wall and each of the retainer arms comprise a lens-contacting surface, wherein the lens-contacting surface of the basket wall is larger than the sum of the lens-contacting surfaces of all retainer arms.

In accordance with another aspect of the carrier according to the invention, the lens-contacting surface of all retainer arms is convexly rounded.

According to still further aspect of the carrier according to the invention, the first concavely curved section of the basket wall has a radius of curvature in the range of 7 mm and 10 mm, in particular in the range of 8 mm and 9 mm. The second concavely curved section of the basket wall has a radius of curvature in the range of 11 mm and 15 mm, in particular in the range of 12 mm and 14 mm. The first portion of the retainer arms having the convex shape has a radius of curvature in the range of 5 mm to 9 mm, in particular in the range of 6 mm to 8 mm. The second portion of the retainer arms having the concave shape has a radius of curvature in the range of 4 mm to 6 mm, in particular in the range of 5 mm to 6 mm.

Another aspect of the invention relates to a carrier array for forming a plurality of carriers as described above. The carrier array comprises a plurality of baskets and a plurality of retainers, wherein each basket of the plurality of baskets comprises a basket wall defining a concave cavity for accommodating an ophthalmic lens. The basket further comprises a plurality of recesses arranged in the basket walls at different angular locations along the circumference of the baskets, each recess of the plurality of recesses being arranged to extend downwardly from an upper rim of the basket wall. Each retainer of the plurality of retainers comprises a plurality of retainer arms corresponds to the plurality of recesses arranged in the basket wall, the retainer arms being arranged in a star-shaped configuration at angular locations corresponding to the angular locations of the recesses along the circumference of the basket. A said retainer is integrally formed with each basket on a side of the basket opposite to the basket wall defining the concave cavity, and the baskets and retainers of the carrier array are arranged in a matrix configuration comprising one or more rows and one or more columns.

A still further aspect of the invention relates to a carrier tray comprising a frame and one or more carrier arrays as described above that are attached to the frame.

Yet a further aspect of the invention relates to a carrier stack system comprising a plurality of carrier trays as described above that are arranged one above the other to form a stack, wherein with respect to two carrier trays adjacently arranged in the stack the retainer arms of the retainer of an upper carrier tray of the two adjacently arranged carrier trays extend into the recesses in the basket wall of the basket of the lower carrier tray of the two adjacently arranged carrier trays.

The carrier according to the present invention - comprising a specially-shaped basket for accommodating an ophthalmic lens and a corresponding specially shaped retainer for retaining the ophthalmic lens in the carrier - advantageously allows for both secure and reliable holding of the ophthalmic lens during its treatment in a processing bath as well as high exposure of the ophthalmic lens to the surrounding treatment liquid. Moreover, the special shape of the carrier promotes a defined position of the ophthalmic lens within the carrier during treatment. The carrier is particularly suitable for the treatment of ophthalmic lenses that are prone to swelling, in particular upon being placed in certain treatment baths, more particular upon being placed in treatment baths containing certain organic solvents (e.g. propanol). In the following, by way of example, contact lenses are discussed as representing a particular type of ophthalmic lenses, without the invention being limited to contact lenses. As used herein, the term "basket" denotes any kind of open receptacle having a cavity defined by a basket wall at least a portion of which is permeable to fluids. That is to say the basket comprises a basket wall having one or more passageways or open areas or perforations or the like such as to allow both liquid and gaseous substances to pass through. As a consequence, treatment liquid may freely flow into and out of the basket which advantageously provides for a continuous flow of treatment liquid over the contact lens accommodated in the carrier. In addition, the permeability of the basket wall to fluids including gaseous substances ensures that air bubbles do not adhere to the contact lens but can freely escape from the carrier during the bath treatment. This may prove advantageous with regard to a high exposure of the contact lens to the surrounding treatment liquid. The form and size of a single passageway or open area or perforation is such as to prevent a contact lens to slip through and thus to escape from the carrier.

In order to allow a sufficiently large amount of treatment liquid to flow into and out of the carrier, the open area percentage of the basket wall should be sufficiently high, possibly at least 30%, 40%, 50%, 60%, 70% or even 80%. As an additional consequence, a high open area percentage provides significant material and weight savings. As used herein, the "open area percentage" is defined as the ratio between the total open area and the total (open and non-open) area of the basket wall. In other words, the open area percentage is a ratio expressed in percent which reflects how much of the total area of the basket wall is formed by passageways or open areas or perforations. On the other hand, the carrier must prevent the contact lens from escaping the carrier, even in case very substantial swelling and subsequent shrinking of the contact lens occurs during the treatment. Also, the carrier prevents that any portion of the contact lens (e.g. the optical zone or the lens edge) may get damaged during swelling and subsequent shrinking. Moreover, at the end of the treatment process the contact lens is arranged in a well-defined position in the carrier for being automatically removed from the carrier with the aid of a gripper, as is well-known in the art.

The basket may comprise a plurality of basket wall segments of a frame-like configuration to minimize flow restrictions for the treatment liquid. The frame-like basket wall segments define the cavity and may support a contact lens accommodated in the basket. For example, the basket may comprise a plurality of V-shaped basket wall segments, with the ends of the arms of the V-shaped basket wall segments being connected by a web, thus forming an enclosed open area.

The curvatures of the first and second concavely curved sections of the basket wall are different, as already mentioned. The difference is in the degree of curvature whereas the type of curvature is the same (i.e. both sections are concavely curved). The curvature within the respective one of the first curved section and the second curved section may be (essentially) the same, i.e. it does not vary within the respective section. Alternatively, it is possible that the curvature may vary within the first concavely curved section and/or within the second concavely curved section. In any event, however, the curvature of the first and second concavely curved sections is different, and is also different at the transition from the first concavely curved section to the second concavely curved section. Thus, it is also possible that the curvature of the first concavely curved section and the curvature of the second concavely curved section are different at the transition, and that at the same time the curvature varies within the first and/or second concavely curved section. To give an example: the curvature of the first concavely curved section may vary between 7 mm and 10 mm (radius of curvature) within the first concavely curved section, while the curvature of the second concavely curved section may vary between 11 mm and 15 mm (radius of curvature) within the second concavely curved section. Despite the variation of the curvature within each of the first and second concavely curved sections, the curvature of the two sections are different from each other even when comparing arbitrary segments of the first and second concavely curved sections. In addition, due to the nonoverlapping ranges of the curvature of the first and second curved sections, the curvatures are also different at the transition.

As mentioned already, the basket is an open receptacle and the basket wall defines an open cavity having a receiving opening defined by an upper rim of the basket wall. A contact lens to be accommodated in the cavity may be inserted into the cavity via the receiving opening. The cavity defined by the basket wall is concave and comprises two different sections that have a different curvature, namely the first concavely curved section and the second concavely curved section, as described above. The curvature of the first concavely curved section, at least in a lower portion thereof, may have a radius of curvature which to some extent corresponds to the radius of curvature of the convex anterior surface of the contact lens to be accommodated in the cavity. Once the contact lens has been inserted into the cavity, the retainer which is used to retain the contact lens in the cavity is that of a further carrier arranged on top. The retainer is brought into engagement with the basket via the receiving orifice. The term "engagement" may or may not comprise a physical contact of the respective parts, or portions thereof, but once the retainer and the basket are in engagement, rotation of the retainer relative to the basket is no longer possible (rotational lock) and the contact lens may not escape from the carrier anymore.

The retainer comprises a plurality of retainer arms which are arranged in a star-shaped configuration at angular locations corresponding to the angular locations of the recesses along the circumference of the basket. Thus, the recesses may serve as guiding means for the retainer arms when the retainer is brought into engagement with the basket to form the afore-described rotational lock.

The carrier may be designed in a manner such that the first concavely curved section is a lower section and the second concavely curved section is an upper section of the basket wall, the upper section and the lower section being a coaxially arranged around a central axis of the basket (i.e. the central axis of rotational symmetry of the basket). Such an arrangement of the carrier is particularly advantageous with respect to providing a well-defined position of the contact lens within the carrier, even during swelling and/or shrinking of the contact lens. Alternatively, this design may be described in a way that the first concavely curved section is an inner section, while the second concavely curved section is an outer section. "Inner" and "outer" may relate to a distance from a central axis of the basket, in particular a central rotational symmetry axis of the basket. This distance is therefore equivalent to a radial distance, and in addition the inner and outer sections are axially offset in the direction of the central symmetry axis of the basket. In particular, the carrier may be designed in such a way that the lower section has a stronger curvature than the upper section. Thus, the contact lens is initially arranged in the lower section, moves to the upper section during swelling, and moves back to the lower section again during shrinking.

If the retainer arms are arranged in a manner such as to have a protruding shape to in the assembled state protrude into the concave cavity defined by the basket wall to further limit the space for the contact lens, a particularly well-defined space for the contact lens may be provided. In particular, the well-defined space further defined by the protruding shape of the retainer arms may be shaped to largely resemble the shape of the contact lens to be contained in the carrier. Thus, a possible deformation, folding or rolling up of the contact lens may be eliminated or at least greatly reduced.

In particular, a design may be used where the protruding shape comprises first and second portions of the retainer arms, the first portion having a convex shape and the second portion having a concave shape. Such a design may provide for a particularly advantageous positioning of the contact lens in the space so formed.

Further, the carrier may be designed in a way that the retainer arms merge radially in a common central hub. The central hub may coincide with the central bottom portion of the basket wall. Thus, a particularly simple and yet particularly mechanically stable and easy to produce design of the carrier may be achieved.

In particular, the design may then easily reflect the basic shape of the contact lens to be contained in the carrier.

If the basket wall comprises a transition section arranged between and connecting the first and second concavely curved sections of the basket wall, this transition section is rounded, so that first and second concavely curved sections are connected with each other without any sharp edges, so that damage to the contact lens can be avoided. In particular, any scratches or the like in the contact lens, particularly in the surfaces of the contact lens, can thus be avoided. Further, during swelling or shrinking of the contact lens such a rounded transition section may enhance sliding of the contact lens over the surfaces of the carrier.

If the basket wall comprises a plurality of basket wall segments and the basket wall segments have a frame-like design as previously described, a carrier with a very high open area percentage can be realised, while at the same time the risk for a contact lens to escape from the carrier is particularly low. In effect, a very large percentage of open area can be realized. Yet, due to the specific design the size of the respective orifices may be comparatively small to avoid the loss of contact lenses during the bath treatment. For example, a number of eight basket wall segments may be particularly advantageous both from the aspect of securely retaining the lens in the carrier during bath treatment while enabling a good flow of liquid into and out of the basket as well as from a manufacturing point of view (injection-molding of the carrier).

If the basket wall and each of the retainer arms comprises a lens contacting surface and the sum of the lens-contacting surfaces of the basket wall is larger than the sum of the lens-contacting surfaces of all retainer arms, the contact lens remains on that surface having the larger overall contact surface at the time the carrier is opened (adhesive effects), i.e. the contact lens remains in the basket in a well-defined position. This allows for an easy removal of the contact lens from the basket in an automated manner, for example using a gripper or other suitable device.

When the lens-contacting surface of all retainer arms is convexly rounded, this may further reduce the contact surface of the retainer arms and also may help avoiding damages to the surfaces of the contact lens. It is to be noted in this regard that the term "convexly rounded" relates to the cross-sectional shape of the respective retainer arm, with the rounded lens-contacting surface of the retainer arms facing towards the contact lens.

The radius of curvature of the first concavely curved section of the basket wall may be in the range of 7 mm and 10 mm, and the radius of curvature of the second concavely curved section of the basket wall may be in the range of 11 mm and 15 mm. The first portion of the retainer arms having the convex shape may have a radius of curvature in the range of 5 mm to 9 mm, and the second portion of the retainer arms having the concave shape may have a radius of curvature in the range of 4 mm to 6 mm. These ranges may be advantageous for soft contact lenses having typical overall lens diameters and curvatures.

The carrier array for forming a plurality of carriers as described above may facilitate the concurrent production of large numbers of contact lenses. Thereby, the manufacturing costs for producing large numbers of contact lenses may be reduced. It is to be noted that the carrier array has the same advantages and features as previously described for the individual carrier in case the individual carriers of the carrier array exhibit the features described above in connection with the individual carrier.

A carrier tray comprising a frame and one or more carrier arrays as described above allows to further increase the efficiency as it enables the simultaneous treatment of a large number of contact lenses, and also allows to easily and quickly attach and detach the carrier arrays to the frame to form the carrier tray. For example, a plurality of carrier arrays can be attached to the frame by means of snap-fit connections so that the carrier trays can be easily assembled and disassembled by 'latching' the carrier arrays to the frame and removing them from the frame by `unlatching' them again. Also, such trays can be arranged one above the other to form a stack. A carrier stack system comprising such a stack of carrier trays allows for a simultaneous treatment of an even larger number of contact lenses. When a carrier stack system comprising such a stack of carrier trays is removed from a bath, it may be tilted relative to the horizontal prior to moving it to the next station in order to avoid liquid to be entrained to the next station, for example to a subsequent bath or a station where unstacking and removal of the contact lenses is performed. During such tilting, the special design of the carrier avoids that the contact lens may slip towards the edge of the basket due to the very limited space defined between the retainer and the basket.

As used in the specification including the appended claims, the singular forms "a", "an", and "other" include the plural, unless the context explicitly dictates otherwise. When using the term "about" with reference to a particular numerical value of a range of values, this is to be understood in the sense that the particular numerical value referred to in connection with the "about" is included and explicitly disclosed, unless the context clearly dictates otherwise. For example, if a range of "about" numerical value a to "about" numerical value b is disclosed, this is to be understood to include and explicitly disclose a range of numerical value a to numerical value b. Also, whenever features are combined with the term "or", the term "or" is to be understood to also include "and" unless it is evident from the specification that the term "or" must be understood as being exclusive.

### BRIEF DESCRIPTION OF THE DRAWINGS

Further advantageous aspects of the invention become apparent from the following description of embodiments of the invention with the aid of the drawings, in which:
- Fig. 1: shows a perspective view from above onto a 4×5 matrix configuration of a carrier array according to one embodiment of the invention, the carrier array comprising a plurality of individual carrier elements;
- Fig. 2: shows a perspective bottom view of the 4×5 matrix configuration of the carrier array of Fig. 1;
- Fig. 3: shows a top perspective view (partially cross-sectioned) of a carrier stack system comprising three carrier trays each comprising a plurality of carrier arrays of Fig. 1 attached to a frame;
- Fig. 4: shows a small section of the carrier stack system of Fig. 3, showing in detail three individual carrier elements arranged one above the other in a stack;
- Fig. 5: shows a cross-section through the three individual carrier elements shown in Fig. 4.

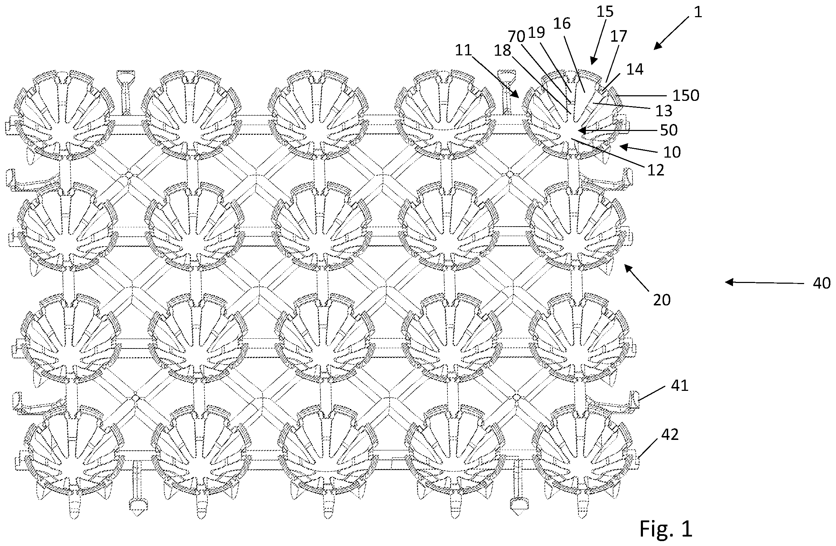

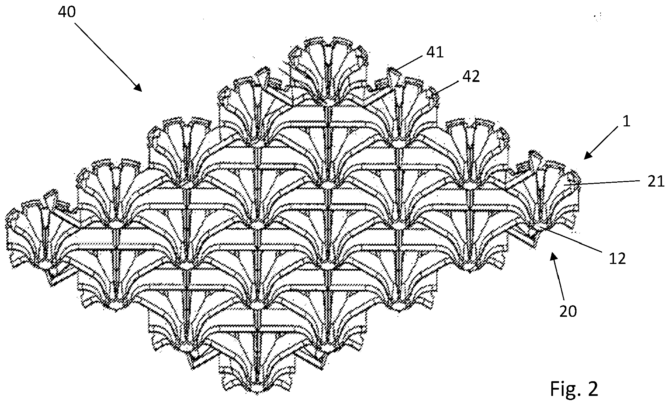

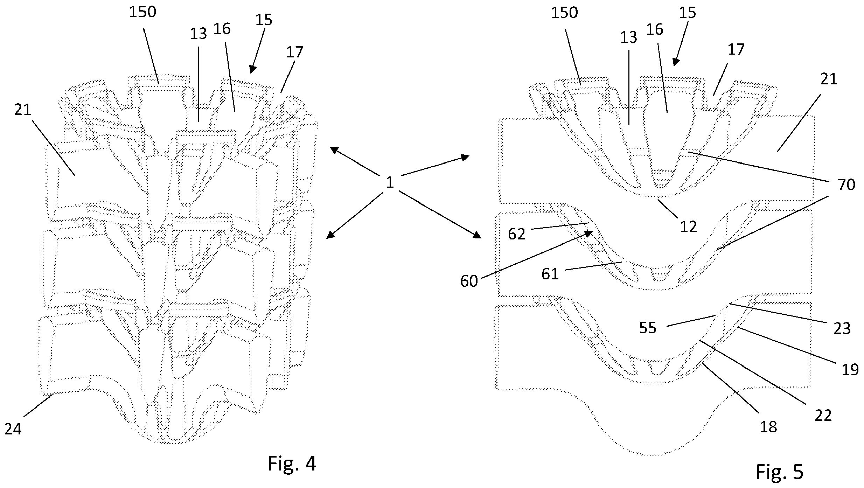

Fig. 1 shows a perspective view of a carrier array 40 comprising a plurality of individual carrier elements 1 arranged in a 4×5 matrix. Each carrier element 1 forms a part of an individual carrier for carrying a contact lens, in particular during a bath treatment. Each individual carrier element 1 comprises a basket 10 (see in particular Fig. 1), the basket wall of which defines a concave cavity 50 for accommodating a contact lens. In addition, each individual carrier element 1 comprises a corresponding retainer 20 (see also Figs. 2 and 4), which is configured to engage with the basket 10 of a further carrier element 1 (not shown in Fig. 1) arranged below the carrier element 1 shown in Fig. 1, to retain the contact lens in the carrier formed by the basket 10 of the further carrier element (not shown in Fig. 1) and the retainer 20 of the carrier element 1 shown in Fig. 1.

Accordingly, in the embodiment shown in Fig. 1 a single individual carrier for carrying a contact lens is formed by the basket 10 of a single carrier element 1 of a further carrier array (not shown in Fig. 1) arranged below the carrier array 40 shown in Fig. 1, and by the retainer 20 of a single carrier element 1 of the carrier array 40 shown in Fig. 1. An individual carrier is also formed by the basket 10 of a single carrier element 1 of the carrier array 40 shown in Fig. 1 and by the retainer 20 of another carrier element 1 of a further carrier array 40 (not shown in Fig. 1) arranged above the carrier array 40 shown in Fig. 1.

Accordingly, in the embodiment shown in Fig. 1, the carrier element 1 may be considered as a constructional base unit having a dual function as being involved in the formation of two adjacent individual carriers, the adjacent individual carriers being arranged one above the other. The basket 10 of the carrier element 1 shown in Fig. 1 serves to accommodate the contact lens to be retained in the carrier by the retainer 20 of a further carrier element 1 (not shown in Fig. 1) arranged above the said basket 10, while the retainer 20 of the carrier element 1 shown in Fig. 1 serves to retain a contact lens accommodated in the basket 10 of a further carrier element 1 arranged below the said retainer 20.

Thus, a single individual carrier element 1 of the embodiment shown comprises both the (concavely shaped) basket 10 and a generally convexly shaped retainer 20. The design here is of a one-piece type, i.e. the carrier element 1 comprises both a basket 10 on its upper side and a retainer 20 on its lower side. Typically, the carrier element 1 is made of a plastic material, e.g. polypropylene (and this holds for the whole carrier array 40, too), so that production of the carrier element 1 or carrier array 40 can be performed using injection molding techniques which are well-known in the art. In the embodiment shown, each basket wall of each basket 10 comprises eight basket wall segments 11 which are circumferentially arranged to form a concave cavity 50. Of course, a different number of basket wall segments 11 can be chosen, for example ten, twelve, or even more. As can be seen in Fig. 1, the basket 10 may have a generally calyx-like configuration and shape, with each of the eight basket wall segments 11 forming a calyx petal. It is to be noted, however, that in the embodiment shown the individual basket wall segments 11 - the individual 'calix petals' - do not form continuous planar surface segments. Instead, originating from a central hub 12 which on one side thereof forms the bottom of the basket wall, a plurality of eight webs 13 emanate in generally radial directions, somewhat similar to the spokes of a wheel. The basket wall segments 11 define the concave cavity 50 for receiving the convex side of a contact lens to be accommodated. At the same time, the rear surfaces of the webs 13 define those portions of the retainer arms 21 of the retainer 20 (see Fig. 2) that hold the contact lens in position in a space 60 (see Fig. 5) formed between the basket wall of the basket 10 of that carrier element 1 (not shown in Fig. 1) which is arranged beneath the carrier element 1 shown in Fig. 1 and by the retainer 20 of the carrier element 1 shown in Fig. 1.

The radial outer end 14 of each of the individual webs 13 is arranged in a recess 17. The recess 17 is formed between two rim portions 150 which are adjacently arranged in circumferential direction. As can be seen in Fig. 1, one rim portion 150 connects two webs 13 which are adjacently arranged in circumferential direction. As can be further seen in Fig. 1, the plurality of presently eight rim portions 150 form a more or less continuous upper rim 15 of the basket, interrupted by the recesses 17.

In the described design, the basket 10 of the embodiment shown in Fig. 1 has eight openings 16, namely one opening 16 in each basket wall segment 11. Thereby, a very large open area percentage can be obtained so that liquid may easily flow into and out of the individual baskets 10, thus enabling an enhanced treatment of the contact lens. At the same time, the openings 16 are still small enough so that there is only a low risk for a contact lens to inadvertently escape from the carrier during bath treatment.

As can be further seen in Fig. 1 (and can be seen even better in Fig. 5), the basket wall comprises a transition section 70. In the embodiment shown, the transition section 70 is arranged essentially halfway along the length of each web 13. The transition section 70 thus separates and connects the first concavely curved section 18 and the second concavely curved section 19 of the basket wall. As already discussed, the radius of curvature is different for the first concavely curved section 18 and the second concavely curved section 19. Namely, the radius of curvature of first concavely curved section 18 is smaller than the radius of curvature of the second concavely curved section 19 (see also Fig. 5). By way of example, in the embodiment shown the radius of curvature of the first concavely curved section 18 may be 8.3 mm (millimeters), while the radius of curvature of the second concavely curved section 19 may be 13 mm. The transition section 70 is embodied such that it connects the first concavely curved section 18 and the second concavely curved section 19 of the basket wall without forming any sharp edges. This allows the contact lens to conveniently move from the first concavely curved section 18 to the second concavely curved section 19 during swelling without getting scratched or otherwise damaged, and also allows the contact lens to conveniently move back from the second concavely curved section 19 to the first concavely curved section 18 during shrinking, from where the contact lens may subsequently be removed with the aid of a gripper.

Thus, the basket wall of each basket 10 comprises a lower section having a stronger curvature (smaller radius of curvature) and an upper section (radially outer section) having a larger radius of curvature which are smoothly connected to one another via the transition section 70.

A transition portion 55 is also provided on the side of the retainer 20 between different portions of the retainer arms 21 (see also Figs. 2, 4, 5). And while the transition section 70 on the side of the basket 10 is a transition section between a first concavely curved section 18 and a second concavely curved section 19 (i.e. both sections connected by the transition section 70 are concavely curved), the transition portion 55 on the side of the retainer 20 defines a changeover from a first portion 22 (inner/lower portion) having a convex shape to a second curved portion 23 (outer/upper portion) having a concave shape, as can be seen best in Fig. 5. By way of example, in the embodiment shown the first portion 22 having the convex shape may have a radius of curvature of 7.2 mm, while the second portion 23 having the concave shape may have a radius of curvature of 5.9 mm.

The transition portion 55 on the side of the retainer 20 - like the transition section 70 on the side of the basket 10 - is embodied such that it connects the first portion 22 of the retainer 20 having the convex shape and the second portion 23 of the retainer 20 having the concave shape without forming any sharp edges. Thus, scratches on the surface of the contact lenses to be treated or other damages can be avoided. Speaking in mathematical terms, this means that the respective curves describing the convex and concave shape are not only continuous but also differentiable in all sections/portions including the transition section 70 and the transition portion 55 and all transitions from one section/portion to another section/portion.

When several carrier elements 1 are placed one above the other, the retainers 20 of a respective upper carrier element 1 and the baskets 10 of a respective adjacently arranged lower carrier element 1 together form the carrier for the contact lens to be treated. First, a contact lens may be placed into the concave cavity 50 defined by the basket wall of the lower carrier element 1, in particular into the concavely curved lower section 18 thereof. Thereafter, the upper carrier element 1 is placed on top of the lower carrier element 1 such that the retainer arms 21 of the retainer 20 of the upper carrier element 1 engage into the recesses 17 arranged in the basket wall of the basket 10 of the lower carrier element 1. For production, a large number of contact lenses may be placed into a large number of such baskets 10, one contact lens into the cavity 50 of each basket 10. This may be automatically performed with the aid of suitable lens grippers, as is known in the art. Subsequently, a corresponding large number of retainers 20 are placed on top of the baskets 10.

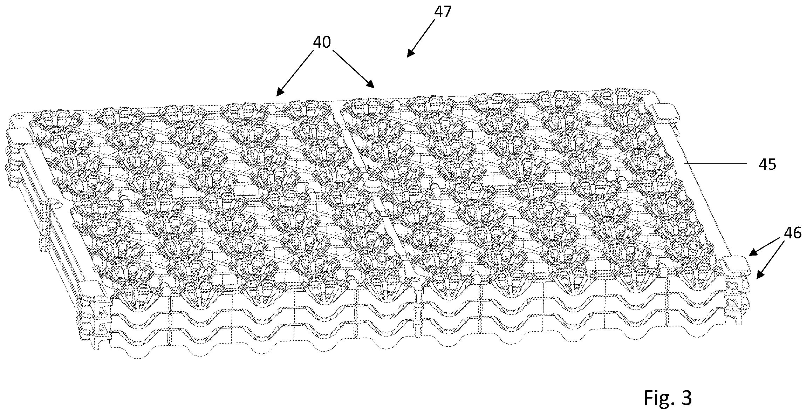

This can be achieved with the aid of carrier arrays 40 as shown in Fig. 1, and in particular it can be achieved with the aid of a plurality of such carrier arrays 40 which are attached to a frame 45 to form a carrier tray 46 as shown in Fig. 3. A plurality of such carrier trays 46 can then be arranged one above the other to form a carrier stack system 47 in which the individual carrier trays 46 are arranged one above the other to form a stack, as also shown in Fig. 3. The thus formed carrier stack system 47 comprising the stack of carrier trays 46 each comprising a plurality of carrier arrays 40 (see Fig. 3) can be handled as a common unit and may be concurrently immersed in a processing bath. It is thus possible to concurrently treat a very large number of contact lenses in the same treatment bath, thereby increasing the efficiency of the production process

In a typical production environment, the contact lenses initially (i.e. after they have been placed into the baskets 10 and the retainers 20 have been placed on top of the baskets 10) do not fill the whole space 60 which is defined between the each basket 10 and the corresponding retainer 20. Instead, typically the contact lenses will take up only a lower portion 61 of the whole space 60. That lower portion 61 may be defined by the lower section 18 of the basket wall of the basket 10 and by the corresponding convex portion 22 of the retainer 20, and initially the contact lens may rest on the lower section 18 of the basket 10.

Once the contact lens swells, for example due to being exposed to certain organic solvents during extraction (see further above), the upper part 62 of the whole space 60 may be partially taken up by the contact lens as well, or even large portions of the contact lens may be arranged in the upper part 62 of the space 60. For example, the contact lens may rest on the upper section 19 of the basket wall of the basket 10 and is thus prevented from folding or rolling up. Also, due to the rotationally symmetric shape of the different sections of the basket 10 (relative to a central symmetry axis of the basket 10) the contact lens is more or less always arranged in a centered manner in the space 60.

As can be seen best from Fig. 4, the lens-contacting surfaces 24 of the retainer arms 21 (i.e. those surfaces facing towards the space 60) are convexly rounded. These rounded lens-contacting surfaces 24 help avoiding the formation of scratches on the surfaces or any other type of damage to the surfaces of the contact lens. In addition, due to being rounded the lens-contacting surfaces 24 comprise only a comparatively small surface area that may actually contact the contact lens. In contrast thereto, the lens-contacting surfaces of the webs 13 (i.e. those surfaces of the webs 13 facing towards the space 60) are more or less planar (see Fig. 5) so that they have a larger surface area when compared to the surface area of the convexly rounded lens-contacting surfaces 24 of the retainer arms 21. This measure assists in making the contact lens adhere to this larger surface area of the surfaces of the webs 13, and ultimately in making the lens remain in the basket 10 when the carrier trays 46 are de-stacked. It is then easily possible to automatically remove the contact lens from the basket 10 with the aid of grippers.

For facilitating the mounting of the carrier arrays 40 to the frame 45 to form the carrier tray 46, and also for the unmounting of the carrier arrays 40 from the said frame 45, each carrier array 40 (see Fig. 1) is provided with a plurality of resilient latches 41 as well as with a plurality of abutments 42, the latches 41 and the abutments 42 being arranged at several locations along the outer edges of the carrier array 40. For mounting the carrier array 40 to the frame 45, a carrier array 40 is inserted into a corresponding opening the frame 45 from below until the abutments 42 engage the lower surface of the frame 45, and until subsequently the latches 41 snap over the upper surface of the frame, thus locking the carrier array 40 to the frame 45. For unmounting the carrier array 40 from the frame, the resilient latches 41 are deflected inwardly and the carrier array 40 is removed from the frame 45 in the reverse order.

As already mentioned, after a plurality of carrier arrays 40 have been mounted to a single frame 45 to form a carrier tray 46, a plurality of such carrier trays 46 can be arranged one above the other in a stack to form a carrier stack system 47 as shown in Fig. 3. Such carrier stack system 47 allows for a very efficient simultaneous treatment of a very large number of contact lenses in a bath.

The present invention has been explained with the aid of an embodiment described with the aid of the drawings. However, the invention is not limited to this embodiment. Instead, various modifications are conceivable for the person skilled in the art without departing from the teaching of the invention. Therefore, the scope of protection is defined by the appended claims.

## Claims

1. Carrier for carrying an ophthalmic lens, in particular a contact lens such as a soft contact lens, during its treatment in a bath, the carrier comprising:
a basket (10) comprising a basket wall defining a concave cavity (50) for accommodating an ophthalmic lens, the basket (10) further comprising a plurality of recesses (17) arranged in the basket wall at different angular locations along the circumference of the basket (10), each recess (17) of the plurality of recesses (17) being arranged to extend downwardly from an upper rim (15) of the basket wall;
and a retainer (20) comprising a plurality of retainer arms (21) corresponding to the plurality of recesses (17) arranged in the basket wall, the retainer arms (21) being arranged in a star-shaped configuration at angular locations corresponding to the angular locations of the recesses (17) along the circumference of the basket (10), to in an assembled state of two such carriers (1) extend into a corresponding one of the plurality of recesses (17) in the basket wall to retain the ophthalmic lens in the carrier,
wherein the basket wall defining the concave cavity (50) comprises first and second concavely curved sections (18, 19), the first and second concavely curved sections (18, 19) each having a curvature, **characterized in that** the curvature of the first concavely curved section (18) is different from the curvature of the second concavely curved section (19).

2. Carrier according to claim 1, wherein the first concavely curved section (18) is a lower section and the second concavely curved section (19) is an upper section of the basket wall, the upper section and the lower section being coaxially arranged about a central axis of the basket (10).

3. Carrier according to claim 2, wherein the lower section has a stronger curvature than the upper section.

4. Carrier according to claim 1, wherein the retainer arms (21) are arranged to have a protruding shape to in the assembled state of two such carriers protrude into the concave cavity (50) defined by the basket wall to further limit a space (60) for the ophthalmic lens in the carrier (1).

5. Carrier according to claim 4, wherein the protruding shape comprises first and second portions (22, 23) of the retainer arms (21), the first portion (22) having a convex shape and the second portion (23) having a concave shape.

6. Carrier according to claim 4, wherein the retainer arms radially merge in a common central hub (12).

7. Carrier according to claim 1, wherein the basket wall comprises a transition section (70) arranged between and connecting the first and second concavely curved sections (18, 19) of the basket wall, wherein the transition section connecting the first and second concavely curved sections (18, 19) is rounded to connect the first and second concavely curved sections (18, 19) without forming any sharp edges.

8. Carrier according to claim 1, wherein the basket wall comprises a plurality of basket wall segments (11), in particular at least eight basket wall segments (11), which are adjacently arranged to one another in the direction of the circumference of the basket (10), each individual basket wall segment (11) of the plurality of basket wall segments (11) being formed by a centrally arranged common basket wall bottom and by two adjacently arranged webs (13) extending radially outwardly from the common basket wall bottom to a radial outer end (14) of the respective web (13), the radial outer end (14) of the respective web (13) being arranged in one of the recesses (17) of the plurality of recesses (17), and the individual basket wall segment (11) further being formed by a rim portion (150) connecting the radial outer ends (14) of the two adjacently arranged webs (13) to define an opening (16) bounded by the common basket wall bottom, the two adjacently arranged webs (13) and the rim portion (150).

9. Carrier according to claim 1, wherein the basket wall and each of the retainer arms (21) comprise a lens-contacting surface (24), and wherein the lens-contacting surface of the basket wall is larger than the sum of the lens-contacting surfaces (24) of all retainer arms (21).

10. Carrier according to claim 9, wherein the lens-contacting surface (24) of all retainer arms (21) is convexly rounded (24).

11. Carrier according to claim 5, wherein the first concavely curved section (18) of the basket wall has a radius of curvature in the range of 7 mm and 10 mm, in particular in the range of 8 mm and 9 mm, and wherein the second concavely curved section (19) of the basket wall has a radius of curvature in the range of 11 mm and 15 mm, in particular in the range of 12 mm and 14 mm, and wherein further the first portion (22) of the retainer arms (21) having the convex shape has a radius of curvature in the range of 5 mm to 9 mm, in particular in the range of 6 mm to 8 mm, and wherein the second portion (23) of the retainer arms (21) having the concave shape has a radius of curvature in the range of 4 mm to 6 mm, in particular in the range of 5 mm to 6 mm.

12. Carrier array (40) for forming a plurality of carriers according to claim 1, the carrier array (40) comprising a plurality of baskets (10) and a plurality of retainers (20),
each basket (10) of the plurality of baskets (10) comprising a basket wall defining a concave cavity (50) for accommodating an ophthalmic lens, the basket (10) further comprising a plurality of recesses (17) arranged in the basket walls at different angular locations along the circumference of the baskets (10), each recess (17) of the plurality of recesses (17) being arranged to extend downwardly from an upper rim (15) of the basket wall; and
each retainer (20) of the plurality of retainers (20) comprising a plurality of retainer arms (21) corresponding to the plurality of recesses (17) arranged in the basket wall, the retainer arms (21) being arranged in a star-shaped configuration at angular locations corresponding to the angular locations of the recesses (17) along the circumference of the basket (10),
wherein a said retainer (20) is integrally formed with each basket (10) on a side of the basket (10) opposite to the basket wall defining the concave cavity (50), and
wherein the baskets (10) and retainers (20) of the carrier array (40) are arranged in a matrix configuration comprising one or more rows and one or more columns.

13. Carrier tray (46) comprising a frame (45) and one or more carrier arrays (40) according to claim 12 attached to the frame (45).

14. Carrier stack system (47) comprising a plurality of carrier trays (46) according to claim 13 arranged one above the other to form a stack, wherein with respect to two carrier trays (46) adjacently arranged in the stack the retainer arms (21) of the retainer of an upper carrier tray (46) of the two adjacently arranged carrier trays (46) extend into the recesses (17) in the basket wall of the basket (10) of the lower carrier tray (46) of the two adjacently arranged carrier trays (46).

## Patentansprüche

1. Träger zum Tragen einer ophthalmischen Linse, insbesondere einer Kontaktlinse, wie einer weichen Kontaktlinse, während deren Behandlung in einem Bad, wobei der Träger umfasst:
einen Korb (10), der eine Korbwand umfasst, die einen konkaven Hohlraum (50) definiert, um eine ophthalmische Linse aufzunehmen, wobei der Korb (10) des Weiteren eine Vielzahl von Aussparungen (17) umfasst, die in der Korbwand in unterschiedlichen Winkelpositionen entlang des Umfangs des Korbs (10) angeordnet ist, wobei jede Aussparung (17) von der Vielzahl der Aussparungen (17) so angeordnet ist, dass sie sich von einer oberen Einfassung (15) der Korbwand abwärts erstreckt;
und einen Halter (20), umfassend eine Vielzahl von Haltearmen (21), die der Vielzahl von Aussparungen (17) entsprechen, die in der Korbwand angeordnet ist, wobei die Haltearme (21) in einer sternförmigen Konfiguration an Winkelpositionen entsprechend den Winkelpositionen der Aussparungen (17) entlang des Umfangs des Korbs (10) angeordnet sind, so dass in einem montierten Zustand von zwei derartigen Trägern (1) diese sich in eine entsprechende von der Vielzahl der Aussparungen (17) in der Korbwand erstrecken, um die ophthalmische Linse in dem Träger zu halten,
wobei die Korbwand, die den konkaven Hohlraum (50) definiert, erste und zweite konkav gekrümmte Segmente (18, 19) umfasst, wobei das erste und das zweite konkav gekrümmte Segment (18, 19) jeweils eine Krümmung aufweist, **dadurch gekennzeichnet, dass** die Krümmung des ersten konkav gekrümmten Segments (18) sich von der Krümmung des zweiten konkav gekrümmten Segments (19) unterscheidet.

2. Träger nach Anspruch 1, wobei das erste konkav gekrümmte Segment (18) ein unteres Segment der Korbwand ist, und das zweite konkav gekrümmte Segment (19) ein oberes Segment der Korbwand ist, wobei das obere Segment und das untere Segment koaxial um eine Zentralachse des Korbs (10) herum angeordnet sind.

3. Träger nach Anspruch 2, wobei das untere Segment eine stärkere Krümmung als das obere Segment hat.

4. Träger nach Anspruch 1, wobei die Haltearme (21) so angeordnet sind, dass sie eine vorstehende Form haben, so dass im montierten Zustand von zwei solchen Trägern diese in den durch die Korbwand definierten konkaven Hohlraum (50) vorstehen, um einen Raum (60) für die ophthalmische Linse in dem Träger (1) weiter einzuschränken.

5. Träger nach Anspruch 4, wobei die vorstehende Form erste und zweite Abschnitte (22, 23) der Haltearme (21) umfasst, wobei der erste Abschnitt (22) eine konvexe Form hat und der zweite Abschnitt (23) eine konkave Form hat.

6. Träger nach Anspruch 4, wobei die Haltearme radial zu einem gemeinsamen zentralen Knotenpunkt (12) zusammengeführt werden.

7. Träger nach Anspruch 1, wobei die Korbwand ein Übergangssegment (70) umfasst, das zwischen dem ersten und dem zweiten konkav gekrümmten Segment (18, 19) der Korbwand angeordnet ist und diese verbindet, wobei das Übergangssegment, welches das erste und das zweite konkav gekrümmte Segment (18, 19) verbindet, gerundet ist, um das erste und zweite konkav gekrümmte Segment (18, 19) zu verbinden, ohne irgendwelche scharfen Ränder zu bilden.

8. Träger nach Anspruch 1, wobei die Korbwand eine Vielzahl von Korbwandsegmenten (11), insbesondere mindestens acht Korbwandsegmente (11), umfasst, die in der Richtung des Umfangs des Korbs (10) benachbart zueinander angeordnet sind, wobei jedes individuelle Korbwandsegment (11) der Vielzahl der Korbwandsegmente (11) durch einen zentral angeordneten gemeinsamen Korbwandboden und durch zwei benachbart angeordnete Bahnen (13) gebildet ist, die sich von dem gemeinsamen Korbwandboden radial auswärts zu einem radialen äußeren Ende (14) der jeweiligen Bahn (13) erstrecken, wobei das radiale äußere Ende (14) der jeweiligen Bahn (13) in einer der Aussparungen (17) der Vielzahl von Aussparungen (17) angeordnet ist, und das individuelle Korbwandsegment (11) des Weiteren durch einen Einfassungsabschnitt (150) gebildet ist, der die radialen äußeren Enden (14) der zwei benachbart angeordneten Bahnen (13) verbindet, um eine Öffnung (16) zu bilden, die durch den gemeinsamen Korbwandboden, die beiden benachbart angeordnete Bahnen (13) und den Einfassungsabschnitt (150) begrenzt ist.

9. Träger nach Anspruch 1, wobei die Korbwand und jeder der Haltearme (21) eine linsenkontaktierende Oberfläche (24) umfassen, und wobei die linsenkontaktierende Oberfläche der Korbwand größer als die Summe der linsenkontaktierenden Oberflächen (24) von allen Haltearmen (21) ist.

10. Träger nach Anspruch 9, wobei die linsenkontaktierende Oberfläche (24) aller Haltearme (21) konvex gerundet (24) ist.

11. Träger nach Anspruch 5, wobei das erste konkav gekrümmte Segment (18) der Korbwand einen Krümmungsradius im Bereich von 7 mm und 10 mm, insbesondere im Bereich von 8 mm und 9 mm hat, und wobei das zweite konkav gekrümmte Segment (19) der Korbwand einen Krümmungsradius im Bereich von 11 mm und 15 mm, insbesondere im Bereich von 12 mm und 14 mm hat, und wobei des Weiteren der erste Abschnitt (22) der Haltearme (21) mit der konvexen Form einen Krümmungsradius im Bereich von 5 mm bis 9 mm, insbesondere im Bereich von 6 mm bis 8 mm hat, und wobei der zweite Abschnitt (23) der Haltearme (21) mit der konkaven Form einen Krümmungsradius im Bereich von 4 mm bis 6 mm, insbesondere im Bereich von 5 mm bis 6 mm hat.

12. Trägergruppierung (40) zum Bilden einer Vielzahl von Trägern gemäß Anspruch 1, wobei die Trägergruppierung (40) eine Vielzahl von Körben (10) und eine Vielzahl von Haltern (20) umfasst,
wobei jeder Korb (10) von der Vielzahl der Körbe (10) eine Korbwand umfasst, die einen konkaven Hohlraum (50) zum Aufnehmen einer ophthalmischen Linse definiert, wobei der Korb (10) des Weiteren eine Vielzahl von Aussparungen (17) umfasst, die in den Korbwänden an unterschiedlichen Winkelpositionen entlang des Umfangs der Körbe (10) angeordnet ist, wobei jede Aussparung (17) von der Vielzahl der Aussparungen (17) so angeordnet ist, dass sie sich von einer oberen Einfassung (15) der Korbwand abwärts erstreckt; und
jeder Halter (20) von der Vielzahl der Halter (20) eine Vielzahl von Haltearmen (21) entsprechend der Vielzahl der Aussparungen (17), die in der Korbwand angeordnet sind, umfasst, wobei die Haltearme (21) in einer sternförmigen Konfiguration in Winkelpositionen entsprechend den Winkelpositionen der Aussparungen (17) entlang des Umfangs des Korbs (10) angeordnet sind,
wobei der Halter (20) innerhalb jedes Korbs (10) auf einer Seite des Korbs (10) gegenüber der Korbwand, die den konkaven Hohlraum (50) definiert, integral gebildet ist, und
wobei die Körbe (10) und Halter (20) der Trägergruppierung (40) in einer Matrixkonfiguration angeordnet sind, die eine oder mehrere Zeilen und eine oder mehrere Spalten umfasst.

13. Trägertablett (46), umfassend einen Rahmen (45) und eine oder mehrere Trägergruppierungen (40) gemäß Anspruch 12, die an dem Rahmen (45) befestigt sind.

14. Trägerstapelsystem (47), umfassend eine Vielzahl von Trägertabletts (46) gemäß Anspruch 13, die eines über dem anderen angeordnet sind, um einen Stapel zu bilden, wobei in Bezug auf zwei Trägertabletts (46), die benachbart in dem Stapel angeordnet sind, die Haltearme (21) des Halters eines oberen Trägertabletts (46) in den zwei benachbart angeordneten Trägertabletts (46) sich in die Aussparungen (17) in der Korbwand des Korbs (10) des unteren Trägertabletts (46) der zwei benachbart angeordneten Trägertabletts (46) erstrecken.

## Revendications

1. Support permettant de transporter une lentille ophtalmique, en particulier une lentille de contact telle qu'une lentille de contact souple, lors de son traitement dans un bain, le support comprenant :
un panier (10) comprenant une paroi de panier définissant une cavité concave (50) destinée à recevoir une lentille ophtalmique, le panier (10) comprenant en outre une pluralité de renfoncements (17) agencés dans la paroi de panier au niveau de différents emplacements angulaires le long de la circonférence du panier (10), chaque renfoncement (17) de la pluralité de renfoncements (17) étant agencé pour s'étendre vers le bas à partir d'un bord supérieur (15) de la paroi de panier ;
et un dispositif de retenue (20) comprenant une pluralité de bras de retenue (21) correspondant à la pluralité de renfoncements (17) agencés dans la paroi de panier, les bras de retenue (21) étant agencés dans une configuration en étoile au niveau d'emplacements angulaires correspondant aux emplacements angulaires des renfoncements (17) le long de la circonférence du panier (10) pour, dans un état assemblé de deux tels supports (1), s'étendre dans un renfoncement correspondant de la pluralité de renfoncements (17) dans la paroi de panier pour retenir la lentille ophtalmique dans le support,
dans lequel la paroi de panier définissant la cavité concave (50) comprend des première et seconde sections incurvées de façon concave (18, 19), les première et seconde sections incurvées de façon concave (18, 19) présentant chacune une courbure, **caractérisée en ce que** la courbure de la première section incurvée de façon concave (18) est différente de la courbure de la seconde section incurvée de façon concave (19).

2. Support selon la revendication 1, dans lequel la première section incurvée de façon concave (18) est une section inférieure et la seconde section incurvée de façon concave (19) est une section supérieure de la paroi de panier, la section supérieure et la section inférieure étant agencées coaxialement autour d'un axe central du panier (10).

3. Support selon la revendication 2, dans lequel la section inférieure présente une courbure plus forte que la section supérieure.

4. Support selon la revendication 1, dans lequel les bras de retenue (21) sont conçus pour avoir une forme saillante pour, à l'état assemblé de deux tels supports, faire saillie dans la cavité concave (50) définie par la paroi de panier pour limiter davantage un espace (60) pour la lentille ophtalmique dans le support (1).

5. Support selon la revendication 4, dans lequel la forme saillante comprend des première et seconde parties (22, 23) des bras de retenue (21), la première partie (22) ayant une forme convexe et la seconde partie (23) ayant une forme concave.

6. Support selon la revendication 4, dans lequel les bras de retenue fusionnent radialement en une base centrale commune (12).

7. Support selon la revendication 1, dans lequel la paroi de panier comprend une section de transition (70) agencée entre et reliant les première et seconde sections incurvées de façon concave (18, 19) de la paroi de panier, dans lequel la section de transition reliant les première et seconde sections incurvées de façon concave (18, 19) est arrondie pour relier les première et seconde sections incurvées de façon concave (18, 19) sans former d'arêtes vives.

8. Support selon la revendication 1, dans lequel la paroi de panier comprend une pluralité de segments de paroi de panier (11), en particulier au moins huit segments de paroi de panier (11), qui sont agencés de manière adjacente les uns aux autres dans la direction de la circonférence du panier (10), chaque segment individuel de paroi de panier (11) de la pluralité de segments de paroi de panier (11) étant formé par un fond de paroi de panier commun agencé centralement et par deux bandes agencées de manière adjacente (13) s'étendant radialement vers l'extérieur à partir du fond de paroi de panier commun jusqu'à une extrémité externe radiale (14) de la bande respective (13), l'extrémité radiale externe (14) de la bande respective (13) étant agencée dans l'un des renfoncements (17) de la pluralité de renfoncements (17), et le segment de paroi de panier individuel (11) étant en outre formé par une partie bord (150) reliant les extrémités radiales externes (14) des deux bandes agencées de manière adjacente (13) pour définir une ouverture (16) délimitée par le fond de paroi de panier commun, les deux bandes agencées de manière adjacente (13) et la partie bord (150).

9. Support selon la revendication 1, dans lequel la paroi de panier et chacun des bras de retenue (21) comprennent une surface de contact avec la lentille (24), et dans lequel la surface de contact avec la lentille de la paroi de panier est plus grande que la somme des surfaces de contact avec la lentille (24) de tous les bras de retenue (21).

10. Support selon la revendication 9, dans lequel la surface de contact avec la lentille (24) de tous les bras de retenue (21) est arrondie de manière convexe (24).

11. Support selon la revendication 5, dans lequel la première section incurvée de façon concave (18) de la paroi de panier a un rayon de courbure dans la plage de 7 mm à 10 mm, en particulier dans la plage de 8 mm à 9 mm, et dans lequel la seconde section incurvée de façon concave (19) de la paroi de panier a un rayon de courbure dans la plage de 11 mm à 15 mm, en particulier dans la plage de 12 mm à 14 mm, et dans lequel en outre la première partie (22) des bras de retenue (21) ayant la forme convexe a un rayon de courbure dans la plage de 5 mm à 9 mm, en particulier dans la plage de 6 mm à 8 mm, et dans lequel la seconde partie (23) des bras de retenue (21) ayant la forme concave a un rayon de courbure dans la plage de 4 mm à 6 mm, en particulier dans la plage de 5 mm à 6 mm.

12. Réseau de supports (40) pour former une pluralité de supports selon la revendication 1, le réseau de supports (40) comprenant une pluralité de paniers (10) et une pluralité de dispositifs de retenue (20),
chaque panier (10) de la pluralité de paniers (10) comprenant une paroi de panier définissant une cavité concave (50) pour accueillir une lentille ophtalmique, le panier (10) comprenant en outre une pluralité de renfoncements (17) agencés dans les parois de panier au niveau de différents emplacements angulaires le long de la circonférence des paniers (10), chaque renfoncement (17) de la pluralité de renfoncements (17) étant agencé pour s'étendre vers le bas à partir d'un bord supérieur (15) de la paroi du panier ; et
chaque dispositif de retenue (20) de la pluralité de dispositifs de retenue (20) comprenant une pluralité de bras de retenue (21) correspondant à la pluralité de renfoncements (17) agencés dans la paroi de panier, les bras de retenue (21) étant agencés dans une configuration en étoile au niveau d'emplacements angulaires correspondant aux emplacements angulaires des renfoncements (17) le long de la circonférence du panier (10),
dans lequel un dit dispositif de retenue (20) est formé d'un seul tenant avec chaque panier (10) sur un côté du panier (10) opposé à la paroi de panier définissant la cavité concave (50), et
dans lequel les paniers (10) et les dispositifs de retenue (20) du réseau de supports (40) sont agencés dans une configuration matricielle comprenant une ou plusieurs rangées et une ou plusieurs colonnes.

13. Plateau de supports (46) comprenant un cadre (45) et un ou plusieurs réseaux de supports (40) selon la revendication 12 fixés au cadre (45).

14. Système de pile de supports (47) comprenant une pluralité de plateaux de supports (46) selon la revendication 13 agencés les uns au-dessus des autres pour former une pile, dans lequel, par rapport à deux plateaux de supports (46) agencés de manière adjacente dans la pile, les bras de retenue (21) du dispositif de retenue d'un plateau de supports supérieur (46) des deux plateaux de supports (46) agencés de manière adjacente s'étendent dans les renfoncements (17) dans la paroi de panier (10) du plateau de supports inférieur (46) des deux plateaux de supports (46) agencés de manière adjacente.
